Europäisches Patentamt

European Patent Office

Office européen des brevets

(11) Publication number: **0 468 340 A2**

## EUROPEAN PATENT APPLICATION

(21) Application number: **91111849.5**

(22) Date of filing: **16.07.91**

(51) Int. Cl.5: **G06F 3/00**

(30) Priority: **24.07.90 US 557205**

(43) Date of publication of application:
**29.01.92 Bulletin 92/05**

(84) Designated Contracting States:
**AT BE CH DE DK ES FR GB GR IT LI LU NL SE**

(71) Applicant: **BioControl Systems, Inc.**
**P.O. Box 2344**
**Stanford, California 94305(US)**

(72) Inventor: **Knapp, Benjamin R.**
**2355 Beach Boulevard, nr 102**
**Pacifica, California 94044(US)**
Inventor: **Lusted, Hugh S.**
**327 Webster Street**
**Palo Alto, California 94301(US)**

(74) Representative: **Baillie, Iain Cameron et al**
**c/o Ladas & Parry, Altheimer Eck 2**
**W-8000 München 2(DE)**

(54) **Eye directed controller.**

(57) A method and apparatus for operating a video game, controlling the cursor position on a computer display, and controlling the operation of equipment and devices using electro-oculogram and electro-myogram signals resulting from eye movement and muscle contractions in the human body. Operator (10) wears sensor assembly (12) which is connected to control processor (18). Movement of the eyes produces electro-oculogram signals which control processor (18) detects and converts into separate control signals representing upward movement, downward movement, movement to the left and movement to the right. Muscle contractions produce electro-myogram signals which control processor (18) detects and converts into control signals. The control signals are interfaced with equipment (22) which is a computer or video game having a display (24), or other apparatus to be controlled. The electro-oculogram control signals are used to control cursor position on a video game or a computer, while the electro-myogram signals are used to simulate the operation of a switch or "fire control" button. The functions of a "joystick", "mouse", "track ball" and the like are thereby replicated by the method and apparatus disclosed herein.

FIG._1

## BACKGROUND OF THE INVENTION

This invention pertains to an eye directed controller generally and more specifically to an apparatus for direct communication with a computer or video game using electro-oculogram (EOG) signals which result from eye movement and electro-myogram signals which result from muscle movement.

Over the past several years interactive video games have gained widespread popularity. Stand-alone game units have been installed in arcades, restaurants, movie theaters, and other publicly accessible facilities. Additionally, video game units have entered the home entertainment market in the form of adapters for standard television sets. These video games require the use of a "joystick," "tracking ball," or other manual input means for playing the game. To play these games proficiently requires considerable "hand-eye" coordination.

Similarly, the use of personal computers has increased both at work and in the home. Many applications which run on personal computers are aided by the use of a "mouse" to control the position of the cursor on the display.

Additionally, a sizeable segment of the handicapped population lacks adequate motor control to operate hand or foot switches, or other types of controls, and require alternative means of operating equipment.

Several types of systems have been developed over the years to permit computers or machinery to be controlled by eye movement alone. For example, U.S. Patent No. 3,462,604 issued to Mason on August 19, 1969, discloses an oculometer for determining eye orientation based on light reflected from the retina of the eye relative to light reflected from the front surface of the eye. Light is directed to the eyeball where it is reflected by both the front surface and the retina at the back of the eye. A detecting system views the reflected images and determines eye position from the relative positions of the reflected images.

U.S. Patent No. 4,109,145 issued to Graf on August 22, 1978, discloses an apparatus for "line of sight" detection of eye orientation. Using an oculometer or other line of sight determining device to monitor the orientation of the eye, the apparatus measures the length of time that the operator's eye remains in the line of sight position relative to a particular control function. If the line of sight position is maintained longer than a predetermined time, the apparatus produces a control output.

U.S. Patent No. 3,986,030 issued to Teltscher on October 12, 1976, discloses an eye-motion operable keyboard accessory for paraplegics or other incapacitated persons. The apparatus includes a light source for directing light toward the operator's eye and a plurality of light responsive sensors which are activated by light reflected from the operator's eye. The sensors are connected to a keyboard actuated instrument to be controlled by the eye movement.

U.S. Patent No. 4,081,623 issued to Vogeley on March 28, 1978, discloses the use of a light source, a radiation sensor, a command discriminator and a display unit to detect when the operator blinks his eyes in a particular coded sequence. The eye blinking is decoded and used to dial a telephone or control a piece of machinery.

U.S. Patent No. 3,507,988 issued to Holmes on April 21, 1970, discloses a narrow-band television system which has resolution characteristics similar to those of the human eye and which is capable of highly resolving a portion of the transmitted field, the location of which is variable in accordance with the line of sight of an observer. Eye position is determined through the use of reflected light.

U.S. Patent No. 3,724,932 issued to Cornsweet et al. on April 3, 1973, discloses an apparatus wherein the eye is flooded with light so that a plurality of Purkinje images are formed by the reflecting surfaces of the eye. Two of the Purkinje images are monitored by imaging them on a rotating disc which has a plurality of orthogonal slits through which the Purkinje images are further imaged on a photodetector. The orientation of the optic axis of the eye is determined by monitoring the separation of the Purkinje images.

U.S. Patent No. 4,866,299 issued to Scharfenberg on September 12, 1989, discloses an apparatus for projecting optical information to a headset so that the wearer can view images which are separate from or superimposed on the directly visible surrounding view.

U.S. Patent No. 4,651,145 issued to Sutter on March 17, 1987, discloses an oculo-encephalographic communication system in which visual stimuli with unique code signals is presented to the operator and the electro-encephalographic (EEG) signal of the operator is monitored. The code can be identified by the EEG signal of the operator and, therefore, a character on a display can be selected simply by looking at it.

U.S. Patent No. 4,576,184 issued to Westerman on March 18, 1986, discloses an apparatus for detecting drug ingestion based upon the corneo-retinal potential and/or brainwaves using an electronystagmograph (ENG) machine.

The disadvantage of many of the systems disclosed in the foregoing patents is that they use light reflected from the eye and are susceptible to interference from other light sources. Other systems require particular visual stimuli to be presented to the operator and corresponding reaction codes to be monitored.

The present invention is preferably less complicated, more reliable and more accurate than the systems disclosed. Quite surprisingly, the EOG signals represent an electrical signature of eye movement and can be used in numerous applications.

See also, Hugh S. Lusted and R. Benjamin Knapp, "Music Produced by Human Bioelectric Signals," presented to the 155th Meeting of the American Association for the Advancement of Science, San Francisco, CA on January 18, 1989.

## SUMMARY OF THE INVENTION

According to one aspect of the invention, there is provided an eye directed controller, comprising means for sensing electro-oculogram signals produced by movement of a human eye; means for processing the electro-oculogram signals into directional control signals representing the direction of the eye movement; and means for interfacing the directional control signals to a device to be controlled.

According to another aspect of the invention, there is provided an eye directed control apparatus, comprising means for sensing electrical signals produced from physiological activity in the body; means for amplifying the electrical signals; means for selectively filtering electro-oculogram signals resulting from eye movement from the amplified electrical signals; means for differentiating between electro-oculogram signals resulting from vertical eye movement and from horizontal eye movement; means for converting the electro-oculogram signals resulting from vertical eye movement into vertical control signals representing upward and downward eye movement; and means for converting the electro-oculogram signals resulting from horizontal eye movement into horizontal control signals representing eye movement in the left direction and eye movement in the right direction.

According to yet a further aspect of the invention, there is provided a method of controlling a device by electrical signals produced by human physiological activity, comprising the steps of sensing electro-oculogram signals produced by eye movement; processing the electro-oculogram signals into directional control signals representing the direction of the eye movement; interfacing the directional control signals to the device to be controlled.

This invention pertains to a new interactive technology that allows direct communication with a computer, video game, or other apparatus simply by using eye movements and/or facial muscle contractions.

The principle behind the eye directed controller disclosed herein is the electro-oculogram (EOG) signal produced by eye movement. An EOG signal is the electrical signature of eye movement. The present invention preferably uses sensors positioned on the head of the operator to detect horizontal and vertical eye movement. The EOG signals are amplified and processed to extract features useful for mapping to output commands, such as controlling the cursor on a computer screen. In this manner, the exact cursor position on a video display may be mapped to a position corresponding to where the eye is focused. The signal processor gives flexibility to the system by allowing various characteristics of the EOG to be mapped to various selectable output commands.

In addition, electro-myogram signals from movement of facial muscles may be utilized to replicate the operation of a switch. For example, double eye blinks can be mapped to a mouse "click," whereas normal single blinks can be ignored. Also, specific eye movement patterns can be programmed to trigger computer functions, such as selecting information in one sector of the video display but not in other sectors.

The invention preferably uses electro-oculogram signals to detect eye movement. The invention may control the cursor position on a computer video display simply by moving the operator's eyes, and provide a computer input device for the disabled.

The invention may also provide an alternative input device for computers, video games and the like, and replicate a computer "mouse" or the like with eye movement.

The invention may replicate a video game "joystick" or like with eye movement, and conveniently increase the skill with which a person can play video games.

## BRIEF DESCRIPTION OF THE DRAWINGS

The invention will be more fully understood by reference to the following drawings which are for illustrative purposes only:

FIG. 1 is a functional diagram of an eye directed controller in accordance with the present invention.

FIG. 2 is a schematic block diagram of the electrical components of the present invention.

FIG. 3 is a schematic block diagram of the instrumentation amplifier element of the apparatus depicted in FIG. 3.

FIG. 4 is a schematic block diagram of the low pass filter element of the apparatus depicted in FIG. 3.

FIG. 5 is a schematic block diagram of the high pass filter element of the apparatus depicted in FIG. 3.

FIG. 6 is a schematic block diagram of the differential comparator of the apparatus depicted in FIG. 3.

FIG. 7 is a schematic block diagram of the single output channel comparator element of the apparatus depicted in FIG. 3.

FIG. 8 is a schematic block diagram of the opto-isolated switch element of the apparatus depicted in FIG. 3.

FIG. 9 is a schematic block diagram of the RMS to DC convertor element of the apparatus depicted in FIG. 3.

FIG. 10 is a schematic block diagram of the electrical components of an alternative embodiment of the present invention.

FIG. 11 is a perspective view of the sensor assembly of the present invention.

## DETAILED DESCRIPTION OF THE PREFERRED EMBODIMENTS

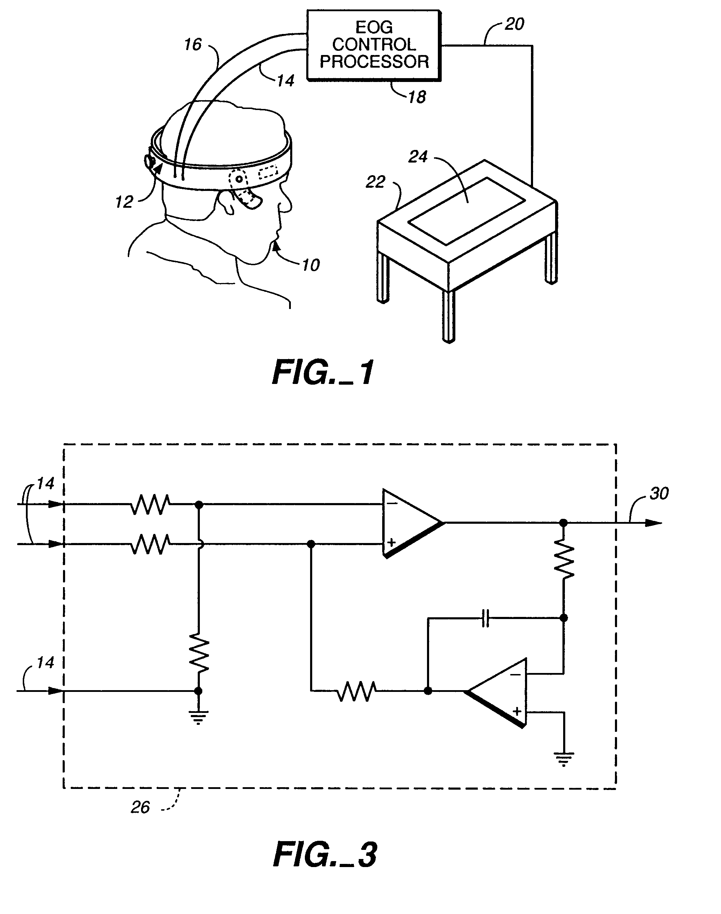

Referring more specifically to the drawings, for illustrative purposes an embodiment of the present invention for use as a "joystick" is generally shown in FIG. 1, FIG. 2 and FIG. 10. It will be appreciated that the apparatus may vary as to configuration and as to details of the parts without departing from the basic concepts as disclosed herein.

An electro-oculogram (EOG) is a record of an electrical trace created by the movement of a human eyeball in relation to the head. The EOG is recorded by placing electrodes around the eyes, detecting the signal produced from eye movement, and amplifying and filtering the electro-physiological signal. To detect vertical movement, electrodes are placed above and below the position of the eyes. To detect horizontal movement, electrodes are placed at the temples.

The EOG signal is created as the eye moves within the electrical field detected by the electrodes. The eye itself acts as a dipole (or battery). The cornea relates to the positive pole while the retina relates to the negative pole.

For example, if electrodes are located symmetrically on either side of the eyes, when an eye is looking straight ahead the dipole is at right angles to the electric field detected by the electrodes and the resultant output is zero. When the eye moves, there exists a voltage shift with reference to the straight ahead position.

Eye movements cause very low frequency (approximately 0.1 Hz to 2 Hz) electrical signals to be generated, in effect producing a time varying shift relative to the steady state output (straight ahead position). The signals are very low in amplitude and must be amplified and filtered to render them useable. Additionally, amplifiers used must have good low frequency response to prevent signal loss.

It is important to note that, to render an EOG signal usable, it is necessary to filter out other physiologically produced signals. For example, an EOG signal is distinguishable from an electro-encephalographic (EEG) signal in amplitude and frequency. An EEG signal is a brainwave signal which is much higher in frequency (approximately 5 Hz to 50 Hz) and arises from large populations of nerve cells in the brain firing synchronously. EEG signals are typically recorded from sensors placed on the scalp and are much lower in amplitude than EOG signals (100 microvolts for EEG contrasted to 1 millivolt for EOG). Thus EOG and EEG signals can be separated by frequency and amplitude.

Electro-myogram (EMG) signals result from muscle movement and vary in intensity depending upon the number of muscle cells participating in a muscle contraction. EMG signals are typically approximately 1 millivolt in amplitude and have a frequency range of approximately 100 Hz to 400 Hz. Therefore, EMG signals can also be separated by frequency and amplitude.

Referring to FIG. 1, a human operator 10 wears sensor assembly 12 on its head. It is significant to note that application of the present invention is not limited to humans, but can be extended to use by animals.

Sensor assembly 12 is connected to EOG control processor 18 through sensor leads 14 and 16. EOG control processor 18 is connected to device 22 through plurality of interconnections 20. Device 22 can be a computer, video game, or other apparatus to be controlled. When operator 10 moves his eyes in any direction, operator 10 can interact with video display 24 or otherwise control device 22 as described herein.

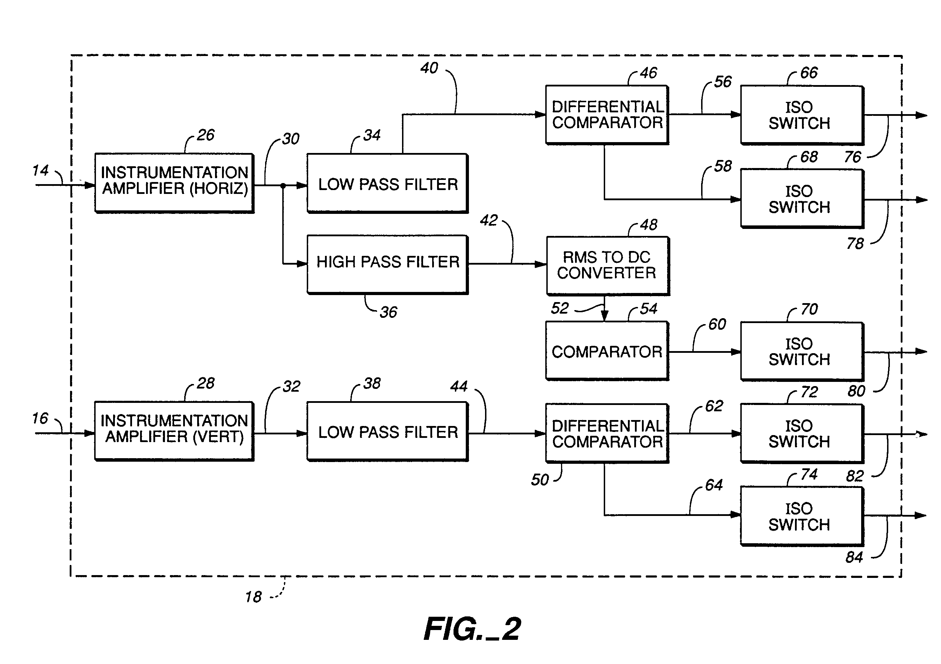

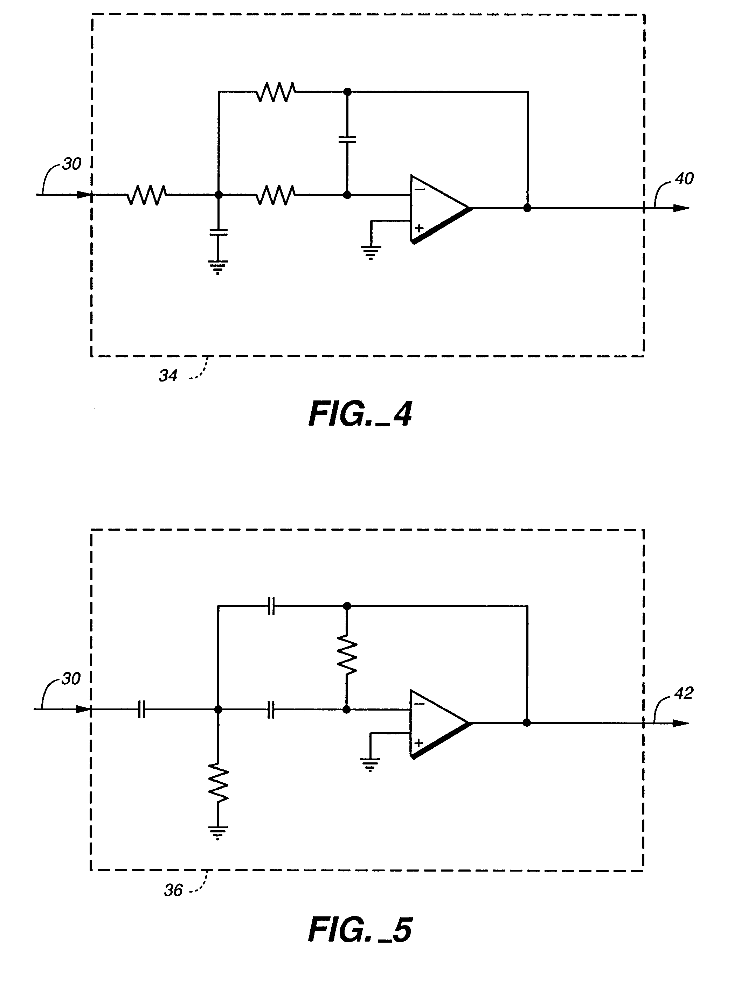

Referring to FIG. 2, instrumentation amplifier 26 amplifies horizontal EOG signals by a factor of approximately 10,000. Instrumentation amplifier 26 is a high impedance, wide bandwidth amplifier with a frequency response down to 0.1 Hz and a noise factor of less than 5 microvolts. Instrumentation amplifier 26 is a differential amplifier and accepts positive and negative horizontal EOG signals from sensor lead 14. FIG. 3 shows a schematic block diagram of instrumentation amplifier 26. Instrumentation amplifier 26 can comprise conventional operational amplifiers such as an LM 363 instrumentation amplifier and discrete components, or their equivalent.

The output of instrumentation amplifier 26 is filtered by low pass filter 34 and by high pass filter 36. Low pass filter 34 is connected to instrumentation amplifier 26 through interconnection 30. High pass filter 36 is also connected to instrumentation amplifier 26 through interconnection 30.

Low pass filter 34 separates the EOG signal from the amplified EEG, EMG and other signals amplified by instrumentation amplifier 26, and passes only the filtered EOG signal to differential comparator 46. Low pass filter 34 has a cutoff frequency of approximately 1.0 Hz and only accepts signals with frequencies below the cutoff point. Low pass filter 34 thereby discriminates and separates the EOG signals from other signals such as EEG and EMG signals which have higher frequencies.

FIG. 4 shows a schematic block diagram of low pass filter 34. Low pass filter 34 can comprise conventional operational amplifiers such as in a TL064 quad device and discrete components, or their equivalent.

High pass filter 36 separates the EMG signal from the amplified EOG, EEG and other signals amplified by instrumentation amplifier 26, and passes only the filtered EMG signal to RMS to DC Converter 48. High pass filter 36 has a cutoff frequency of approximately 100.0 Hz and only accepts signals with frequencies above the cutoff point. High pass filter 36 thereby discriminates and separates EMG signals produced from muscle contractions from EOG and EEG signals which have lower frequencies. The EMG signal can then be used as a switch similar to pushing a button or clicking a key on a mouse or other device.

FIG. 5 shows a schematic block diagram of high pass filter 36. High pass filter 36 can comprise conventional operational amplifiers such as in a TL064 quad device and discrete components, or their equivalent.

Differential comparator 46 is interconnected to low pass filter 34 through interconnection 40. Differential comparator 46 compares the amplified and filtered horizontal EOG signal with a positive and negative reference voltage. If the amplitude of the EOG signal exceeds the positive reference voltage, differential comparator 46 activates iso switch 66. If the amplitude of the EOG signal is more negative than the negative reference voltage, differential comparator 46 activates iso switch 68. This results in direct correlation between the direction of eye gaze to the left or right and joystick operation.

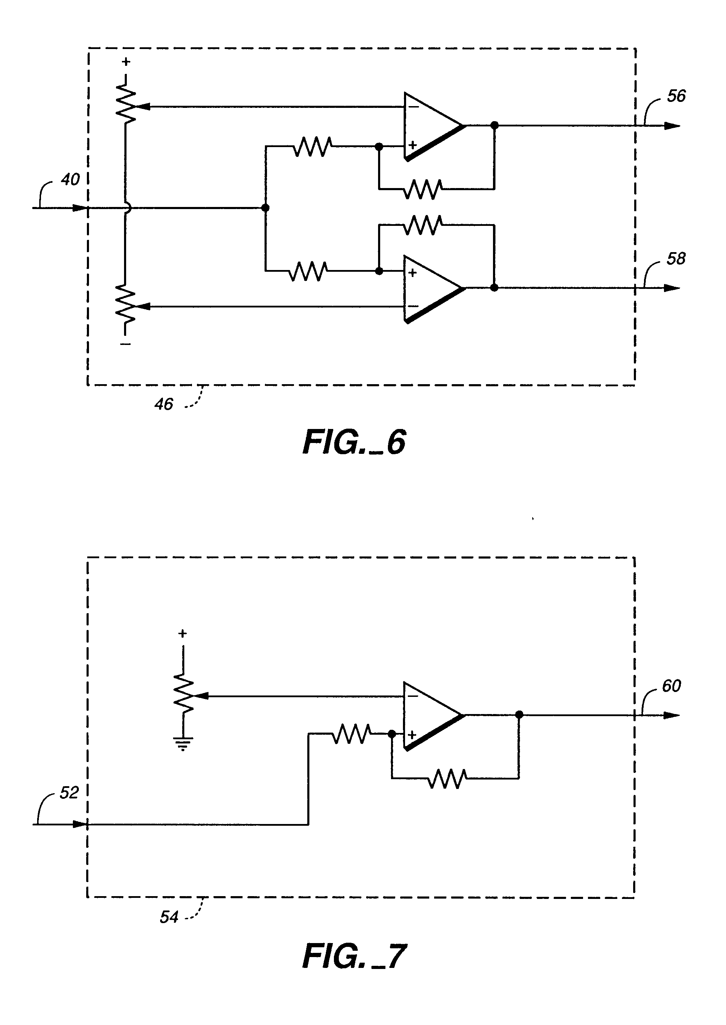

FIG. 6 shows a schematic block diagram of differential comparator 46. Differential comparator 46 comprises a differential comparator having positive and negative reference voltages to distinguish between left and right eye movements, and two output channels to differentiate between left and right eye movement.

Differential comparator 46 serves to compare the amplitude of the EOG signals with a preset threshold level so that signals from "stray" eye movements are ignored and only signals corresponding to intentional eye movements are converted to control signals.

Differential comparator 46 can comprise conventional operational amplifiers such as in a TL064 quad device and discrete components, or their equivalent.

RMS to DC convertor 48 is connected to high pass filter 36 through interconnection 42, and creates a voltage trigger level in response to the EMG signal resulting from muscle movement. RMS to DC convertor 48 integrates the EMG signal over a period of approximately 1.0 seconds and produces an amplified DC output signal proportional to the energy associated with the EMG signal.

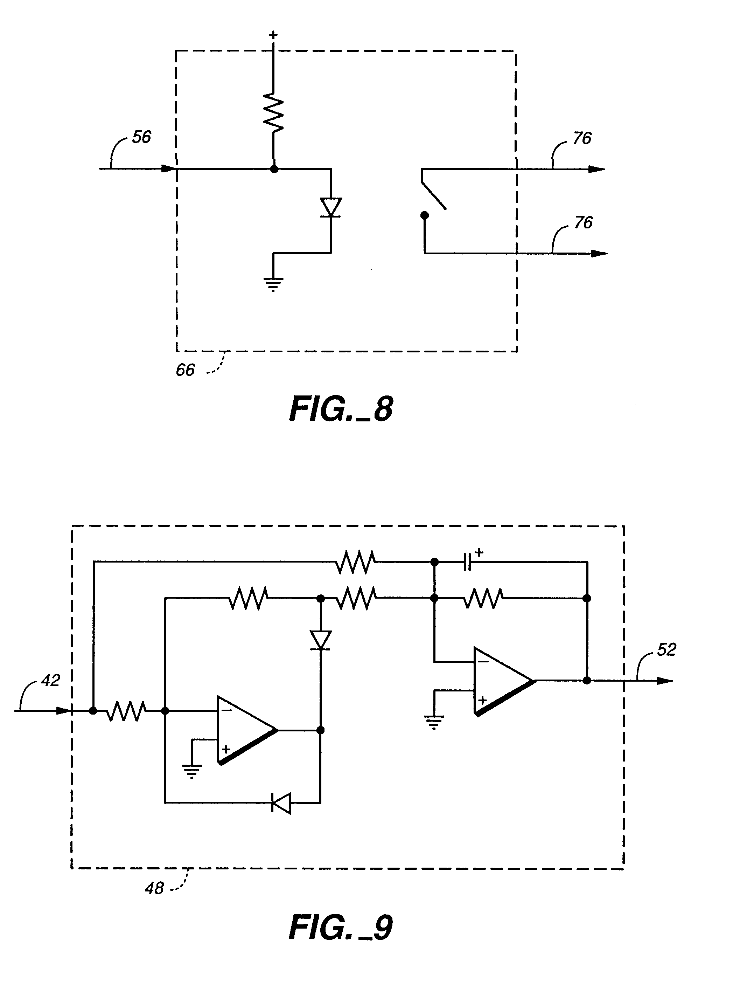

FIG. 9 shows a schematic block diagram of RMS to DC convertor 48. RMS to DC convertor 48 can comprise conventional operational amplifiers such as in a TL064 quad device and discrete components, or their equivalent.

The output of RMS to DC convertor 48 is connected to comparator 54 through interconnection 52. Comparator 54 compares the EMG signal to a predetermined reference voltage, and outputs a +5 volt signal if the EMG signal exceeds the reference voltage. In this manner, the EMG signal resulting from a muscle "jerk" or other significant movement of the muscles about the head can be used as a "trigger" control or other equivalent to pushing a button or activating a switch on the device to be operated. Stray muscle movements are ignored and only EMG signals from intentional movements are converted to control signals.

FIG. 7 shows a schematic block diagram of comparator 54. Comparator 54 can comprise conventional operational amplifiers such as in a TL064 quad device and discrete components, or their equivalent.

Vertical EOG signals from sensor lead 16 are amplified, filtered and processed in a manner similar to that for horizontal signals. The difference is that EMG signals are not separated and processed from the vertical input channel. Instrumentation amplifier 28 is the same in structure and operation as instrumentation amplifier 26 and is connected to low pass filter 38 through interconnection 32. Low pass filter 38 is the same in structure and operation as low pass filter 34 and is connected to differential comparator 50 through interconnection 44. Differential comparator 50 is the same in structure and operation as differential comparator 46.

Iso switch 66 is connected to differential comparator 46 through interconnection 56 and serves as a switch responding to the left eye movement signal from differential comparator 46.

Iso switch 68 is connected to differential comparator 46 through interconnection 58 and serves as a switch responding to the right eye movement signal from differential comparator 46.

Iso switch 70 is connected to comparator 54 through interconnection 60 and serves as a switch responding to muscle "jerks" or other significant muscle movement.

Iso switch 72 is connected to differential comparator 50 through interconnection 62 and serves as a switch responding to upward eye movement.

Iso switch 74 is connected to differential comparator 50 through interconnection 64 and serves as a switch responding to downward eye movement.

The plurality of iso switches 66, 68, 72 and 74 replicate the function of a "joystick" for video games, thereby permitting the user to move an object in two dimensions to any point in a video screen. The control signals from the iso switches can also be used to control the cursor position on a computer display. Each switch is activated from a 5 volt signal coming from differential comparator 46 or differential comparator 50. Iso switches 66, 68, 72 and 74 are connected to the equipment to be controlled through interconnections 76, 78, 82 and 84, respectively.

Iso switch 70, which is activated by a muscle "jerk", is connected to the game input port to serve the function of selector button for the game. For example, a "fire control" button for an aerial combat game. Iso switch 70 is connected to the equipment to be controlled through interconnection 80.

Referring to FIG. 8, each iso switch is optically isolated from the electrical input of the computer, video game, or other apparatus to be controlled. Each iso switch can be a single optical isolated switch in a quad device such as an HSR-8200 or equivalent, or comprise discrete components or their equivalent.

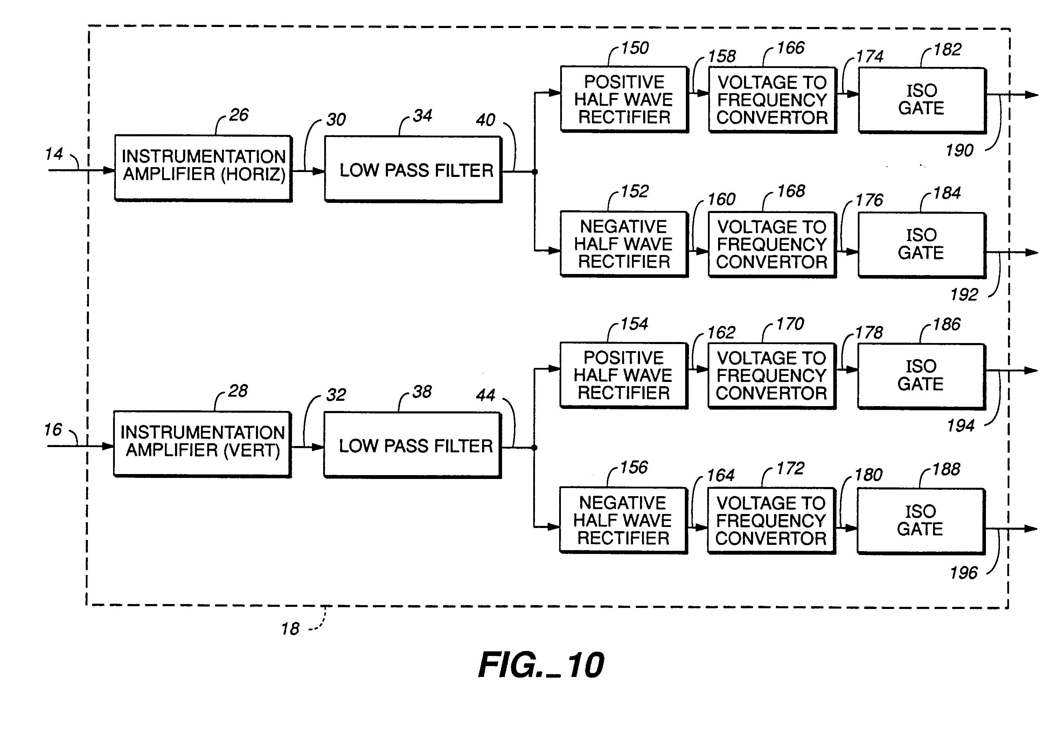

A computer "mouse" can be replicated by incorporating a frequency to voltage convertor in the circuit as shown in FIG. 10. A mouse differs from a joystick in that a joystick closes switches corresponding to "left," "right," "up," and "down" movement whereas a mouse produces a sequence of pulses corresponding to "left," "right," "up," and "down" movement. The amount of movement in a given direction is reflected in the number of pulses produced.

Referring to FIG. 10, the circuit for a mouse differs from that for a joystick in the processing of the amplified and filtered EOG signals. For horizontal movement, the EOG signal from low pass filter 34 is split into a positive direct current voltage component by positive half wave rectifier 150 and a negative direct current voltage component by negative half wave rectifier 152. A positive voltage from positive half wave rectifier 150 represents movement in the right direction. A negative voltage from negative half wave rectifier 152 represents movement in the left direction.

Positive half wave rectifier 150 and negative half wave rectifier 152 are connected to the output of low pass filter 24 through common interconnection 40.

The positive output voltage from positive half wave rectifier 150 is converted into pulses by voltage to frequency convertor 166 which is connected to the output of positive half wave rectifier 150 through interconnection 158. Voltage to frequency convertor 166 produces a stream of pulses which have a frequency directly proportional to the positive voltage level from positive half wave rectifier 150. As the voltage increases, the frequency and therefore, the number of pulses per given time unit, increases. The number of pulses produced by voltage to frequency convertor 166 represents the amount of movement in the right direction.

Voltage to frequency convertor 166 can be an LM 331 and discrete components, or their equivalent.

The pulses produced by voltage to frequency convertor 166 then drive iso gate 182 which is connected to voltage to frequency convertor 166 through interconnection 174. Iso gate 183 serves as an optically isolated gate to interface with the serial input of a computer or other device, and can be an HP 6N138 or equivalent. Iso gate 183 is connected to the serial input of a computer or other device through interconnection 190.

For movement in the left direction, the negative output voltage from negative half wave rectifier 152 is converted into pulses by voltage to frequency convertor 168 which is connected to the output of negative half wave rectifier 152 through interconnection 160. Voltage to frequency convertor 168 is identical to voltage to frequency convertor 166 except that its input contains an invertor to convert the negative voltage from negative half wave rectifier 152 into a positive voltage suitable for driving the voltage to frequency convertor.

When the eyes are straight ahead, there is no voltage differential sensed by instrumentation amplifier 26 and the output of positive half wave rectifier 150 and the output of negative half wave rectifier 152 are both zero. Movement in the right direction with reference to center (looking straight ahead) produces a positive voltage whereas movement in the left direction produces a negative voltage. Therefore, the pulses produced represent movement in only one direction at a time.

Vertical eye movement is converted to pulses in the same manner as for horizontal movement, and it can be seen that the vertical movement portion of the circuit shown in FIG. 10 is identical to the horizontal movement portion of the circuit shown in FIG. 10 in all respects.

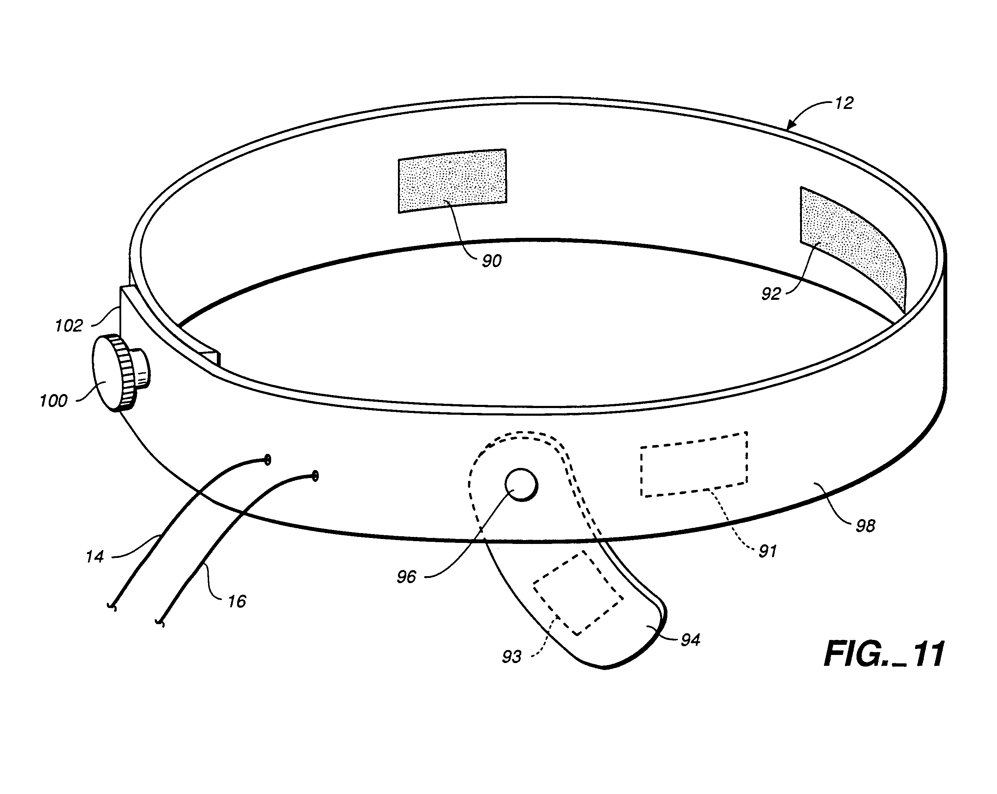

Referring to FIG. 11, sensor assembly 12 is connected to EOG control processor 18 through interconnections to input channels 14 and 16. Sensor assembly 12 generally comprises a head band 98 made of leather, plastic or other semi-rigid material which wraps around the head of operator 10. The fitting of head band 98 is adjusted by an adjusting knob 100. Adjusting knob 100 controls the position of adjusting band 102 relative to head band 98. By turning adjusting knob 100, the tightness of head band 98 on operator 10 can be varied. It should be appreciated that there are many other means for attaching sensor assembly 12 to operator 10 and adjusting it to fit operator 10.

Sensor assembly 12 contains horizontal input electrode 90, dual input electrode 91, reference electrode 92, and vertical input electrode 93. Dual input electrode 91 serves as both a horizontal input and vertical input electrode. These electrodes are typically made of silver mesh cloth or other suitable flexible conductive material.

Electrodes 90 and 91 are positioned laterally opposed to each other on sensor assembly 12 such that they will make contact to operator 10 in proximity to the temples of the head when sensor assembly 12 is worn by operator 10.

Reference electrode 92 is positioned on sensor assembly 12 such that it will make contact with the forehead of operator 10.

Vertical electrode 93 is positioned on cheekplate 94. Cheekplate 94 is attached to sensor assembly 12 by means of fastener 96. Fastener 96 can be an eyelet or other fastener which would permit cheekplate 94 to swivel about the axis at the connection point to sensor assembly 12. Cheekplate 94 is adjusted to position its vertical electrode 93 in proximity to the cheek of operator 10.

Sensor lead 14 comprises three individual wires, one of which is connected to horizontal electrode 90 positioned near the left temple of operator 10, one of which is connected to reference electrode 92 positioned near the forehead of operator 10, and the last of which is connected to dual input electrode 91 positioned near the right temple of operator 10.

Sensor lead 16 also comprises three individual wires, one of which is connected to dual input electrode 91, one of which is connected to reference electrode 92, and the last of which is connected to vertical electrode 93 mounted on cheekplate 94 and positioned near the cheek of operator 10.

It can be seen, that sensor leads 14 and 16 each have a wire which is used as a common connection to reference electrode 92. Horizontal EOG and vertical EOG signals are measured using that electrode as a reference point.

Sensor leads 14 and 16 are connected to the inputs of instrumentation amplifier 26 and 28, respectively.

Accordingly, by means of electrical signals produced by eye movements or muscle contractions it will be seen that the present invention can be used to direct the position of a cursor on a computer video display, replicate the function of a "joystick" for video games, serve as "mouse" for a computer, and operate other types of equipment and machinery. Although the description above contains many specificities, these should not be construed as limiting the scope of the invention but as merely providing illustrations of some of the presently preferred embodiments of this invention. Thus the scope of this invention should be determined by the appended claims and their legal equivalents.

**Claims**

1. An eye directed controller, comprising:
    (a) means for sensing electro-oculogram signals produced by movement of a human eye;
    (b) means for processing said electro-oculogram signals into directional control signals representing the direction of said eye movement; and
    (c) means for interfacing said directional control signals to a device to be controlled.

2. The apparatus as recited in claim 1, further comprising:
    (a) means for sensing electro-myogram signals produced by muscle movement;
    (b) means for processing said electro-myogram signals into trigger control signals representing said muscle movement; and
    (c) means for interfacing said trigger control signals to said device to be controlled.

3. The apparatus as recited in claims 1 or 2, wherein said means for sensing electro-oculogram signals comprises a plurality of electrodes.

4. The apparatus as recited in claim 3, wherein said plurality of electrodes comprises:
    (a) an electrode positioned in proximity to the forehead of a human operator;
    (b) an electrode positioned in proximity to a facial cheek of said human operator;
    (c) an electrode positioned in proximity to the right temple of said human operator; and
    (d) an electrode positioned in proximity to the left temple of said human operator.

5. The apparatus as recited in claim 3, wherein said electrodes are silver mesh cloth.

6. The apparatus as recited in claims 1 to 5, wherein said directional control signals are switch closures.

7. The apparatus as recited in claim 2, wherein said trigger control signal is a switch closure.

8. The apparatus as recited in claim 7, wherein said device to be controlled is a video game.

9. The apparatus as recited in claims 1 to 5, wherein said directional control signals are pulses.

10. The apparatus as recited in claim 9, wherein said device to be controlled is a computer.

11. The apparatus as recited in claims 1 to 10, further comprising a low pass filter, said low pass filter having a cutoff frequency below 5.0 Hertz.

12. The apparatus as recited in claim 11, further comprising means for differentiating between said electro-oculogram signals produced from vertical eye movement and horizontal eye movement.

13. The apparatus as recited in claim 12, further comprising:
(a) means for differentiating between electro-oculogram signals produced from horizontal eye movement in the left direction and electro-oculogram signals produced from horizontal eye movement in the right direction; and
(b) means for differentiating between electro-oculogram signals produced from upward eye movement and electro-oculogram signals produced from downward eye movement.

14. The apparatus as recited in claim 2, further comprising a high pass filter having a cutoff frequency above 50.0 Hertz.

15. An eye directed control apparatus, comprising:
(a) means for sensing electrical signals produced from physiological activity in the body;
(b) means for amplifying said electrical signals;
(c) means for selectively filtering electro-oculogram signals resulting from eye movement from said amplified electrical signals;
(d) means for differentiating between electro-oculogram signals resulting from vertical eye movement and from horizontal eye movement;
(e) means for converting said electro-oculogram signals resulting from vertical eye movement into vertical control signals representing upward and downward eye movement; and
(f) means for converting said electro-oculogram signals resulting from horizontal eye movement into horizontal control signals representing eye movement in the left direction and eye movement in the right direction.

16. The apparatus as recited in claim 15, wherein said means for selectively filtering said electro-oculogram signals from said amplified electrical signals comprises a low pass filter having a cutoff frequency below 5.0 Hertz.

17. The apparatus as recited in claim 16, wherein said means for converting said electro-oculogram signals resulting from vertical eye movement into said vertical control signals comprises a differential comparator.

18. The apparatus as recited in claim 17, wherein said means for converting said electro-oculogram signals resulting from horizontal eye movement into the horizontal control signals comprises a differential comparator.

19. The apparatus as recited in claims 15 to 18, further comprising:
(a) means for selectively filtering electro-myogram signals resulting from muscle movement from said amplified electrical signals; and
(b) means for converting said electro-myogram signals resulting from said muscle movement into trigger control signals.

20. The apparatus as recited in claim 19, wherein said control signals are switch closures.

21. The apparatus as recited in claims 15 to 19, wherein said control signals are pulses.

22. The apparatus as recited in claim 19, wherein said means for selectively filtering electro-myogram signals resulting from muscle movement from said amplified electrical signals comprises a high pass filter having a cutoff frequency above 50.0 Hertz.

23. The apparatus as recited in claim 22, wherein said means for converting said electro-myogram signals into said trigger control signals comprises:
(a) an RMS to DC convertor; and
(b) a comparator.

24. A method of controlling a device by electrical signals produced by human physiological ac-

tivity, comprising the steps of:

(a) sensing electro-oculogram signals produced by eye movement;

(b) processing said electro-oculogram signals into directional control signals representing the direction of said eye movement;

(c) interfacing said directional control signals to the device to be controlled.

25. The method as recited in claim 24, further comprising the steps of:

(a) sensing electro-myogram signals produced by muscle movement;

(b) processing said electro-myogram signals into trigger control signals; and

(c) interfacing said trigger control signals to the device to be controlled.

**FIG._1**

**FIG._3**

FIG._2

EP 0 468 340 A2

*FIG._4*

*FIG._5*

**FIG._6**

**FIG._7**

**FIG._8**

**FIG._9**

**FIG._10**

EP 0 468 340 A2

FIG._11

EP 0 468 340 A2